# EUROPEAN PATENT APPLICATION

(11) **EP 2 105 148 A1**
(43) Date of publication of application: **30.09.2009**
(21) Application number: 08153647.6
(22) Date of filing: 28.03.2008
(51) Int. Cl.: A61L 9/12, A61L 9/22

(54) **An air conditioner**

(71) Applicant: Sara Lee/DE N.V., 3532 AA Utrecht (NL)
(72) Inventor: Albos, Barbara, 08017 Barcelona (ES); Ortiz, Marc, 08020 Barcelona (ES); Garcia, Mario, 08940 Cornellà de Llobregat (ES)
(74) Representative: Hatzmann, Martin

(57) **Abstract**

The invention relates to an air conditioner 10 comprising an inlet and an outlet, an air displacement unit 2 arranged to generate air flow in a pathway between the inlet and the outlet, an electrostatic filter 1 arranged in the pathway for intercepting particle contamination in the air flow and a fluid delivery unit 5 comprising a fluid, said fluid delivery system being arranged at least in a vicinity of the pathway downstream the filter to cooperate with the air displacement unit for enhancing discharge of the fluid towards the outlet.

## Description

### FIELD

The invention relates to an air conditioner arranged to purify and to add a suitable fluid to an air flow.

### BACKGROUND

An embodiment of an air conditioner as is set forth in the foregoing is known from WO 2005/079875. The known air conditioner constitutes an integrated device arranged to combine the functions of air purifier and volatile liquid disseminator. For this purpose the known air conditioner comprises a fan arranged to generate an air flow in a suitable pathway between an inlet and an outlet of the device and a wetted wick arranged in a cross-section of said pathway. The wetted wick has one end immersed in the volatile liquid arranged in a reservoir and the other end arranged in the cross-section of the pathway. The air flow generated in the known air conditioner is purified using a fibrous filter and an electrically heatable catalyst monolith.

It is a disadvantage of the known air conditioner that in order to purify air complex means are provided, in particular means than consume substantial amount of energy. In addition, due to provision of a number of substantially massive bodies being spanned over the whole cross-section of the air pathway, only air flow of a limited strength can be generated post filter and catalyst monolith, which limits a degree of evaporation of the volatile liquid from the wick. Still additionally, due to the fact that in the known air conditioner either the air pathway or the wick are to be bend, the efficacy of the fluid pick-up by the air flow is decreased.

### SUMMARY OF THE INVENTION

It is an object of the invention to provide an air conditioner wherein air purification function is effectively combined with fluid dissemination function. It is a further object of the invention to provide an air conditioner wherein a rate of the fluid pick-up by the air flow is increased. It is a still further object of the invention to provide an air conditioner having limited power consumption.

To this end the air conditioner according to the invention comprises:
- an inlet and an outlet;
- an air displacement unit arranged to generate air flow in a pathway between the inlet and the outlet;
- an electrostatically chargeable filter arranged in the pathway for intercepting particle contamination in the air flow;
- a fluid delivery unit comprising a fluid, said fluid delivery system being arranged at least in a vicinity of the pathway downstream the filter to cooperate with the air displacement unit for enhancing discharge of the fluid towards the outlet.

The technical measure of the invention is based on the insight that, first, by providing an electrostatically chargeable filter particular contamination can effectively be eliminated from the incoming air flow. It has been found that electrostatic filters are capable of intercepting particles with dimensions in the range of at least 0.3 to 10 micrometers. Because particles of small dimensions are intercepted by the filter, an inner volume of the air conditioner is substantially particle-free, which improves the overall functioning of the device due to the fact that no obstruction of a surface conceived for enabling the fluid evaporation is formed by dust particles which may be otherwise accumulated on the surface of the fluid delivery system.

Due to the fact that the filter acts on ionized particles, it does not have to be massive or voluminous. As a result, the electrostatic filter does not substantially affect the power of the air stream generated by the air displacement unit in the air conditioner. It will be appreciated that the electrical power consumption of the electrostatically chargeable filters is orders of magnitude smaller than a power consumption of a heatable catalyst monolith.

Secondly, due to the fact that the fluid delivery system is arranged at least in a vicinity of the pathway downstream in a vicinity of the air displacement unit, the air flow generated by the air displacement unit effectively enhances discharge of the fluid towards the outlet. It will be appreciated that for the air displacement unit a suitable air blower or a suitable fan may be selected, although these examples are not limitative.

It will further be appreciated that due to the fact that an air flow having a substantial flow power may be generated inside the air conditioner unit due to absence of massive parts obstructing the air athway, it is sufficient to place the fluid discharge system just in a suitable vicinity of the thus generated air flow. For volatile liquids, like perfumes, the air flow will pick up sufficient amount of perfume molecules escaping a perfume vessel and conduct them to the outlet of the air conditioner.

In an embodiment of the air conditioner according to the invention, the fluid delivery unit comprises a vessel mountable inside the air conditioner.

It is found to be particularly advantageous to position the fluid delivery system inside the air conditioner. In this case a suitable vessel comprising the volatile fluid may be positioned in a direct vicinity of the air displacement unit, wherein power of the air flow is maximal, which increases an evaporation degree and a air flow pick-up level of the fluid. Preferably, for such positioning a suitable projection on the vessel may be envisaged. Alternatively, the vessel may be mountable using its bottle neck, for example the vessel may be hanged by its bottle neck on a suitable clamp close to an air exit surface of the air displacement unit. For example, in an embodiment of the air conditioner according to the invention, the air displacement unit may comprise a blade which is rotatably arranged with respect to a stator, the fluid delivery system being positioned in a direct vicinity of the stator. Preferably, the fluid delivery system is substantially dimensioned as the stator. In this way, the vessel of the fluid delivery system is positioned in an area of a maximal air flow power, yet substantially not obstructing the air flow. This embodiment achieves a maximum evaporation and pick-up rate while keeping the air conditioner ergonomic and simple in construction.

In a further embodiment of the air conditioner according to the invention, the fluid delivery system comprises a wettable wick.

It is found to be advantageous to conduct the volatile fluid to an area of the substantial air flow. This can simply be achieved by means of a wettable wick, having a first end positioned inside a fluid vessel in contact with the fluid, and a second end positioned in the air flow. Preferably, the wick is substantially straight and is manufactured from a single piece of porous material. By avoiding bends or assemblies of parts in the wick a homogenous structure is provided with respect to pore sizes having no interfaces, which improves fluid conduction along the wick towards the area where molecules of the volatile fluid are conceived to be picked-up by the air flow. As a result, the evaporation rate of the volatile fluid is further increased.

In a further embodiment of the air conditioner according to the invention, it is provided with a cover comprising cooperating constituents, at least one of said constituents being arranged to at least partially support the filter.

It is found to be advantageous to at least partially house the filter in a cover of the air conditioner according to the invention. In this way a compact and ergonomic apparatus is provided.

In a further embodiment of the air conditioner according to the invention, a tuner may be provided for adjusting the air flow generated by the air displacement unit.

Due to the fact that the air conditioner according to the invention is capable of generating an air flow of substantial power yet consuming a small amount of electrical power, it is found to be advantageous to allow for adjustment of said power. It will be appreciated that, in particular, in domestic applications, the air conditioner may be used to provide a little amount of background odor for enhancing pleasuring domestic atmosphere. Alternatively, when a large volume of air has to be purified and perfumed, for example in kitchen or post smoking in a room, the air flow power may be set to a maximum level for efficiently removing unpleasant and/or potentially hazardous matter from the atmosphere. It will be appreciated that a great plurality of possible embodiments of the tuner is possible, including a continuous tuner from a minimum to a maximum power level, or a step-wise regulator covering a number of dwell positions within the envisaged power range. The tuner may also be implemented as a suitable timer, which may advantageously be arranged to alternate fragrance discharge with no fragrance discharge. This will result in a provision of interrupted flow of fragrance, which will improve perception of the discharged fragrance due to absence of habituation of user's receptors.

In a further embodiment of the air conditioner according to the invention, the fluid delivery system comprises an indicator for indicating an amount of the fluid in the fluid delivery system.

It is found to be advantageous to provide a suitable indicator for feed-backing actual level of the fluid inside the fluid delivery system. The indicator may be implemented as a visual means, for example by providing a transparent fluid vessel through which a user may observe the level of the fluid inside the vessel. In order to ease such examination, the cover of the air conditioning unit may be provided with an inspection window through which the vessel is visible. In particular, the inspection window may be provided with a lens focusing on the vessel. Alternatively, the inspection window may be provided with a mirror directed to the vessel. Still alternatively, the indicator may be arranged as electric means comprising two electrodes conceived to be positioned on or near the bottom of the vessel for measuring electrical resistance there between. It will be appreciated that when the fluid is emptied, the resistance will increase, which is indicative of the level of the fluid inside the vessel. It is possible that the electrodes are connected with a light signaling means, for example LED, for indicating that the fluid discharge system may be refilled.

In a further embodiment of the air conditioner according to the invention an electrical safety switch is provided for disconnecting an electrical power supply to at least the air displacement unit upon a disengagement of the cooperating constituents of the cover.

It is found to be advantageous to provide one or more safety switches integrated in the cover of the air conditioner, in particular for disconnecting the air displacement unit from the means when the cover is opened. This feature improves the electrical safety of the apparatus.

These and other aspects of the air conditioner according to the invention will be discussed in more detail with reference to drawings, wherein like numerals denote like elements. It will be appreciated that the drawings are provided for illustrative purposes only and may not be used to limit a scope of the appended claims.

### BRIEF DESCRIPTION OF THE DRAWINGS

Figure 1 presents a schematic elevated view of an embodiment of an air conditioner according to the invention.
Figure 2 presents a further schematic view of an embodiment of an air conditioner according to the invention.

### DETAILED DESCRIPTION OF THE DRAWINGS

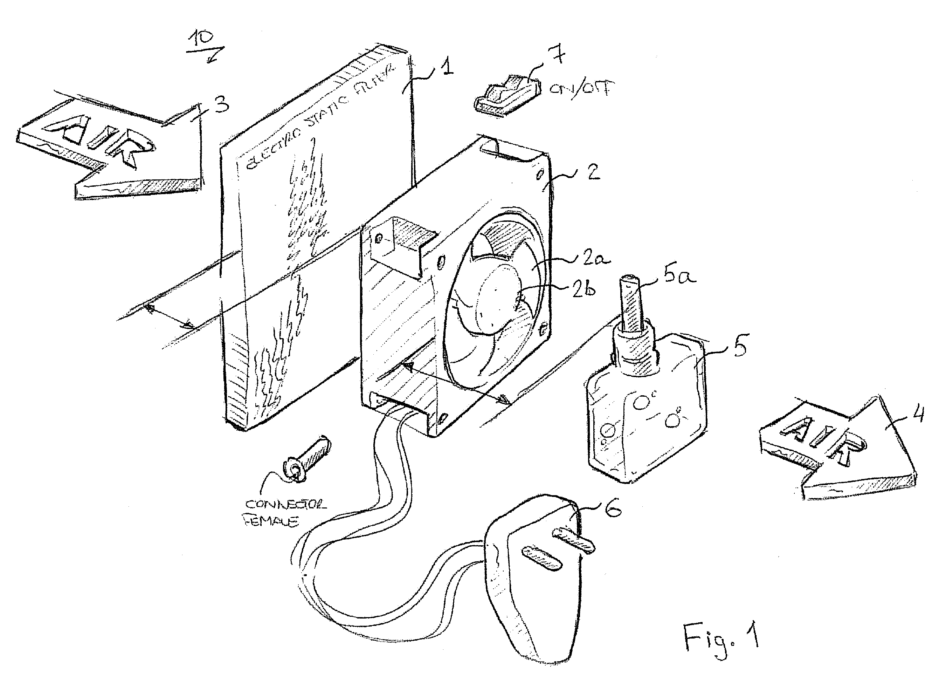

Figure 1 presents a schematic elevated view of an embodiment of an air conditioner 10 according to the invention. The air conditioner 10 comprises an air displacement unit 2, for example, a fan having one or more rotatable blades 2a and a stator 2b. The air displacement unit is arranged to generate an incoming air flow 3 from the environment of the air conditioner. The air flow 3 enters an inner volume of the air conditioner 10 using a suitable inlet (not shown) which may comprise one or more suitably shaped openings. The air conditioner 10 comprises an electrostatic filter 1 arranged to intercept the incoming air flow 3 and to collect particular contamination having a size at least in the range of 0.3 - 10 micrometers. The air displacement unit 2 may be powered from the mains using a suitable power connector 6. The cable may be between 1m and 3m of length and may be connectable to the air conditioner via a jack connector. It will be appreciated that the air conditioner 10 may also be powered using batteries. The engine of the fan 2 may be located in the stator 2b and may be provided with a suitable adaptor for converting an input voltage (220 V) into a suitable output voltage (12V).

The purified air exits the air displacement unit 2 and collects molecules of a suitable volatile fluid from a wick 5a or directly from a suitable vessel of a fluid discharge system 5 arranged in a direct vicinity of the air flow just exiting the air displacement unit 2. The purified air flow 4 provided with the molecules of the fluid exits the air conditioned via an outlet (not shown) into a suitable environment, for example a room. The air conditioner 10 is further provided with an ON/OFF button 7 and a tuner (not shown) for adjusting a power of the air flow 3, 4.

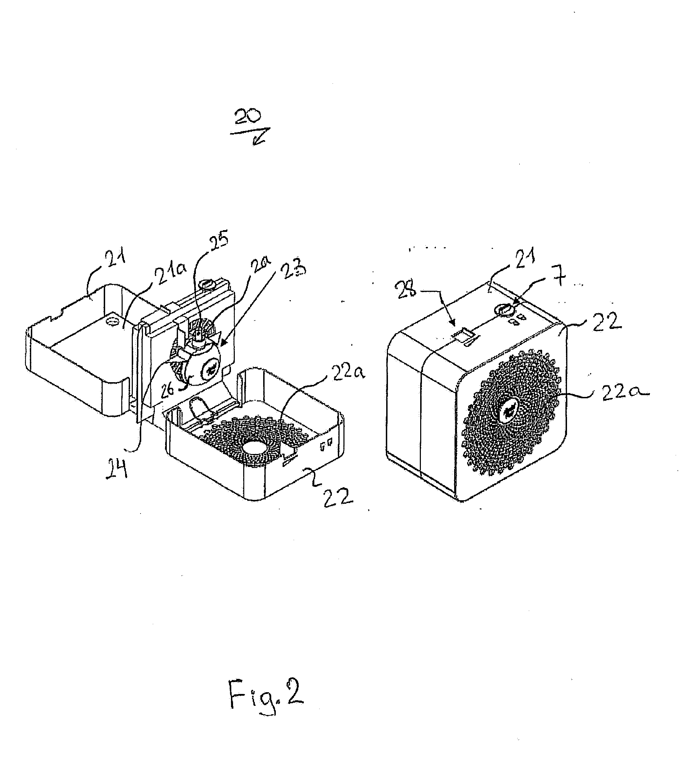

The air conditioner 10 may further comprise a printed circuit board (PCB) for enabling electrical connectivity to the ON/OFF switch, the connector to the electrical cable and one or more safety switches, which may be linked to any of the plastic covers (shown in Figure 2) of the air conditioner. The safety switch may be arranged to disconnect electrical power supply to the air displacement unit 2 upon an event one of the covers is open while the device is operating.

It will be appreciated that due to the fact that the fluid discharge system 5 is positioned inside the air conditioner 10 in a direct vicinity of the air flow generated by the air displacement unit 2, both the air pathway 3 - 4 and the wick 5a may be kept straight which, first, leads to absence of losses of air flow power due to undesirable bends in the pathway, and, secondly, absence of losses of capillary power due to undesirable bends in the wick and/or use of two wicks attached to each other to form a 90 degree bend. In addition, due to positioning of the fluid discharge system inside the air conditioner close to the area of maximum air flow, no wick may be necessary.

Figure 2 (left portion) presents a further schematic view of an embodiment of an air conditioner according to the invention. The air conditioner 20 comprises two cooperating cover elements 21, 22, which may be manufactured from plastic. The covers 21, 22 may be arranged to protect an internal plastic structure 23 which may locate an engine (not shown) of the air displacement unit and a fluid discharge system 26.

The cover 21 may be shaped to at least partially support and/or house the electrostatic filter 21a. The filtered air flow is directed towards the fluid discharge system 26.

The fluid discharge system 26 may be provided as a suitable vessel having a bottle neck, wherein the bottle neck is used for mounting the vessel 26 substantially at a position of the stator of the fan by using suitably arranged clamps 24. The one or more blades 2a of the air displacement unit generate the air flow which comes into immediate contact with the vessel substantially in region of a highest flow power thereby increasing an evaporation rate of the fluid stored in the vessel. The vessel may be provided with a wick 25, or without it.

The cover 22 may be provided with one or more suitably shaped outlets 22a for discharging the purified air which is provided with a suitable fluid. Examples of the fluid are perfumes, medicine, insecticide or a fungicide.

Figure 2 (right portion) presents an embodiment of the air conditioner according to the invention when the cover constituents 21, 22 are put together. The air conditioner may be switched on using an ON/OFF button 7, whereby power of the generated air flow may be adjusted using a tuner 28.

For operating the air conditioner unit 20 the user may plug the adaptor (not shown) to the mains and may connect the cable to the device, for example by means of a jack connector. To refill the device the user may disengage covers 21, 22 in order to locate the filter in its suitable place, indicated by the frame and a fluid discharge system, for example, a fragrance bottle. The fragrance bottle may be hanged by the bottle neck in a suitable plastic clamp close to the fan outlet. It is preferable to provide the filter and the fluid discharge system which may be used during a time period of about 2 months. It is preferable to provide a refill comprising both the filter and a new vessel.

While specific embodiments have been described above, it will be appreciated that the invention may be practiced otherwise than as described. The descriptions above are intended to be illustrative, not limiting. Thus, it will be apparent to one skilled in the art that modifications may be made to the invention as described in the foregoing without departing from the scope of the claims set out below.

## Claims

1. An air conditioner comprising:
- an inlet and an outlet;
- an air displacement unit arranged to generate air flow in a pathway between the inlet and the outlet;
- an electrostatic filter arranged in the pathway for intercepting particle contamination in the air flow;
- a fluid delivery unit comprising a fluid, said fluid delivery system being arranged at least in a vicinity of the pathway downstream the filter to cooperate with the air displacement unit for enhancing discharge of the fluid towards the outlet.

2. An air conditioner according to claim 1, wherein the fluid delivery unit comprises a vessel mountable inside the air conditioner.

3. An air conditioner according to claim 2, wherein the vessel comprises a bottle having a bottle neck, the vessel being mountable using the bottle neck.

4. An air conditioner according to any one of the preceding claims, wherein the air displacement unit comprises a blade which is rotatably arranged with respect to a stator, the fluid delivery system being positioned in a direct vicinity of the stator.

5. An air conditioner according to claim 4, wherein the fluid delivery system is substantially dimensioned as the stator.

6. An air conditioner according to claim 4 or 5, wherein, the fluid delivery system comprises a wettable wick.

7. An air conditioner according to claim 6, wherein the pathway and the wick are straight.

8. An air conditioner according to any one of the preceding claims, provided with a cover comprising cooperating constituents, at least one of said constituents being arranged to at least partially support the filter.

9. An air conditioner according to any one of the preceding claims, further comprising a tuner for adjusting the air flow generated by the air displacement unit.

10. An air conditioner according to any one of the preceding claims, wherein the fluid delivery system comprises an indicator for indicating an amount of the fluid in the fluid delivery system.

11. An air conditioner according to any of the preceding claims, further comprising an electrical safety switch for disconnecting an electrical power supply to at least the air displacement unit upon a disengagement of the cooperating constituents of the cover.
